# EUROPÄISCHE PATENTSCHRIFT

(11) **EP 3 205 722 B1**
(45) Veröffentlichungstag und Bekanntmachung des Hinweises auf die Patenterteilung: **01.08.2018**
(21) Anmeldenummer: 16155286.4
(22) Anmeldetag: 11.02.2016
(51) Int. Cl.: C12N 15/10

(54) **VORRICHTUNG UND VERFAHREN ZUR ISOLIERUNG VON NUKLEINSÄUREN AUS VOLLBLUT**
DEVICE AND METHOD FOR INSULATING NUCLEIC ACIDS FROM WHOLE BLOOD
DISPOSITIF ET PROCEDE D'ISOLEMENT D'ACIDES NUCLEIDES DANS LE SANG TOTAL

(43) Veröffentlichungstag der Anmeldung: 16.08.2017
(73) Patentinhaber: Sarstedt AG & Co. KG, 51588 Nürnbrecht (DE)
(72) Erfinder: Schuster, Rainer, 51582 Nümbrecht (DE)
(74) Vertreter: Taruttis, Stefan Georg

(56) Entgegenhaltungen:
- WO-A1-00/09746
- WO-A1-2006/052680
- WO-A1-2007/060248
- WO-A1-2009/018034
- CN-A- 103 820 431
- CN-A- 104 673 623
- DE-A1- 10 147 439
- DE-B3-102014 220 090
- US-A1- 2016 032 277

## Beschreibung

Die vorliegende Erfindung betrifft die Verwendung einer Zusammensetzung und ein Verfahren zur Stabilisierung von RNA, einschließlich der in Zellen enthaltenen RNA, aus Vollblut, bevorzugt mit der anschließenden Isolierung der RNA. Eine Vorrichtung zur Verwendung als Blutentnahmeröhre, die die zur Stabilisierung der gesamten RNA, und die für die anschließende Isolierung geeignete Zusammensetzung enthält, wird ebenfalls beschrieben. Die Zusammensetzung zur Stabilisierung der RNA aus Vollblut, das Verfahren und die für das Verfahren verwendbare Blutentnahmeröhre, die die Zusammensetzung enthält, zeichnen sich dadurch aus, dass eine effektive Stabilisierung von RNA in Vollblut erreicht wird und ein schnelles und effektives Verfahren zur Isolierung ermöglicht. Dabei umfasst die Stabilisierung die gesamte RNA, einschließlich der im zellfreien Plasma enthaltenen RNA, und die in den im Blut vorliegenden Zellen enthaltene RNA.

### Stand der Technik

Die WO2009/018034 A1 betrifft die Steigerung der Löslichkeit von SDS in Lysepuffer für Zellen durch Zusatz eines nichtionischen Detergenzes und ein Verfahren zur Isolierung von DNA aus tierischem Gewebe mit Zugabe eines Lysepuffers aus 2M NaCl, 1,2% SDS, 12mM EDTA, 24mM Tris-HCl, pH 8,0 mit 2% Tween und anschließender Zugabe des zehnfachen Volumens eines Extraktionspuffers aus 50mM Tris-HCl, pH 7, 10mM EDTA, 7M Guanidin-HCl, 5% Tween20.

Die WO 2007/060248 A1 richtet sich auf die Lyse von Zellen mit chaotropem Salz und anschließender Zugabe von nicht chaotropem Salz zur Bindung von Nukleinsäuren an Trägermaterial. Zur Isolierung von DNA aus Lebergewebe wird z.B. ein Lysepuffer aus 4M Guanidinthiocyanat, 1% N-Laurylsarcosin und 2mM EDTA mit anschließender Kontaktierung der Mischung mit Glasfasermaterial zur Adsorption der DNA beschrieben.

Die CN 103820431 A beschreibt nach der englischen Zusammenfassung keine Zusammensetzung zur Lyse von Zellen.

Die DE 101 47 439 A1 beschreibt zur Isolierung von DNA aus Blut, dass ein Lysereagenz zugegeben wird und anschließend durch Zentrifugation DNA-haltige Zellkomponenten abgetrennt werden. Die sedimentierte DNA wird durch Resuspendieren mit Guanidiniumhydrochlorid und Abtrennen von Verunreinigungen, z.B. von Protein, gereinigt und durch Zugeben von Alkohol gefällt und abgetrennt.

Die DE 10 2014 220 090 B3 beschreibt zum Sammeln nukleinsäurehaltiger biologischer Proben ein Gefäß, in das ein Abstrichtupfer eingebracht werden kann, wobei das Gefäß eine Lyseflüssigkeit enthält.

Die CN 104673623 A beschreibt nach der englischen Zusammenfassung für ein Probengefäß mit Deckel eine Zusammensetzung zur Stabilisierung von Hämatozyten-Nukleinsäuren und von zellfreier DNA.

Die WO 2006/052680 A1 beschreibt die Isolierung von Nukleinsäuren aus Blutproben mit einem Verfahren, bei dem zunächst durch Zentrifugation ein Pellet abgeschieden wird, das von Blutbestandteilen durch Waschen gereinigt wird und anschließend nochmals zu einem Pellet abzentrifugiert wird. Anschließend wurden Pellets in Lysepuffer aus 6 M LiCl, 5% Triton X-100, 5% DGME, 10 mM EDTA, 100 mM TRIZMA, pH 8,8, mit Zusatz von Lösungshilfsmitteln (38 mM TRIZMA Base, 12 mM TRIZMA HCl, 10 mM EDTA, 5% Triton X-100) gelöst, optional durch Zusatz von weiterem Triton X-100. Anschließend wurde diese Mischung mit dem Adsorptionsmittel für Nukleinsäuren kontaktiert.

Die US 2016/0032277 A1 beschreibt zur Isolierung von microRNAs ein Verfahren, bei dem zunächst eine Phenol-Chloroform-Extraktion vorgenommen wird und anschließend die wässrige Phase abzentrifugiert wird und mit einem Träger für Nukleinsäuren kontaktiert wird. Als Lysepuffer kann eine wässrige Mischung aus Detergenz, z.B. Triton oder Tween, Guanidin-HCl, EDTA und TRIS verwendet werden, die auch frei von Reduktionsmitteln (β-Mercaptoethanol) sein kann.

Die WO02/056030 A2 beschreibt zur Stabilisierung des Gehalts an RNA in einer Probe einen Behälter mit vorbestimmtem Vakuum zum Einziehen eines vorbestimmten Probenvolumens, wobei in dem Behälter ein Mittel zur Hemmung der Geninduktion und gegen den enzymatischen Abbau von Nukleinsäuren vorgelegt ist, insbesondere Mercaptoethanol, Dithiotreitol (DTT) und ein chaotropes Salz, insbesondere Guanidiniumisothiocyanat oder Guanidiniumhydrochlorid.

Die WO 00/09746 A1 beschreibt ein Gefäß zur Blutentnahme, das zur Lyse von Zellen und zur Stabilisierung von Nukleinsäuren eine Lösung mit einem Guanidiniumsalz, Puffer, Detergenz und einem Reduktionsmittel, insbesondere DTT, β-Mercaptoethanol und TCEP (Tris(2-Carboxyethyl)phosphin) enthält. Das Reduktionsmittel, insbesondere β-Mercaptoethanol oder DTT, wird darin als wesentlich für die Stabilisierung von RNA in Serum angesehen.

### Aufgabe der Erfindung

Der Erfindung stellt sich die Aufgabe, eine alternative Verwendung der Zusammensetzung und bevorzugt ein alternatives Verfahren zur Stabilisierung und anschließenden Isolierung der gesamten RNA aus Vollblut bereitzustellen. Bevorzugt soll die Zusammensetzung die gesamte RNA aus Vollblut stabilisieren und ein schnelles Verfahren zur Isolierung der gesamten RNA erlauben. Weiter bevorzugt soll die Zusammensetzung die Stabilisierung der RNA für ein variables Blutvolumen erlauben, so dass eine Blutentnahmeröhre, die die Zusammensetzung enthält, nicht notwendigerweise eingerichtet sein muss, ein vorbestimmtes Blutvolumen einzuziehen.

### Beschreibung der Erfindung

Die Erfindung löst die Aufgabe mit den Merkmalen der Ansprüche, insbesondere durch die Verwendung einer Zusammensetzung zur Stabilisierung der gesamten RNA, und ein Verfahren zur Stabilisierung der gesamten RNA aus Vollblut zur anschließenden Isolierung und dient gleichzeitig zum Aufschluss von Zellen, so dass zelluläre Nukleinsäuren in dem Zustand bei der Kontaktierung mit der Zusammensetzung,
insbesondere zum Zeitpunkt der Blutentnahme, freigesetzt und hinsichtlich Beschaffenheit und Konzentration stabil erhalten werden und anschließend isoliert werden können. Dabei zeichnet sich die Zusammensetzung dadurch aus, dass sie insbesondere den Abbau und die Neusynthese von RNA in Vollblut verhindert, in das sie eingemischt ist. Entsprechend betrifft die Erfindung die Verwendung der Zusammensetzung, bevorzugt enthalten in einer Blutentnahmeröhre, für das Verfahren, insbesondere als Mittel zur Stabilisierung und Isolierung der Gesamt-RNA aus Blut ohne den Schritt des Ausfällens und Abtrennens von RNA vor Adsorption der RNA an ein Adsorptionsmittel. Zur Isolierung kann die Mischung aus Blut und der wässrigen Zusammensetzung ohne Fällung der RNA mit einem Adsorptionsmittel kontaktiert werden. Die Isolierung der RNA durch Kontaktieren der Mischung aus der wässrigen Zusammensetzung und Blut mit einem Adsorptionsmittel kann unmittelbar aus der vollständigen Mischung der wässrigen Zusammensetzung mit Blut erfolgen, d.h. z.B. ohne Fällung und Abtrennung von Nukleinsäuren aus der Mischung, optional nach einer Lagerung, während derer die Mischung z.B. vom Ort der Blutentnahme zum Ort des Analyseverfahrens transportiert wird.

Die biologische Probe ist Vollblut, auch als Blut bezeichnet.

Die Zusammensetzung weist in wässriger Lösung
zumindest ein Guanidiniumsalz, bevorzugt Guanidiniumthiocyanat,
zumindest eine Puffersubstanz, bevorzugt 2-(*N*-Morpholino)ethansulfonsäure (MES),
ein nichtionisches Detergenz, bevorzugt Triton X-100 und
einen Komplexbildner für zweiwertige Kationen, bevorzugt Ethylendiamintetraacetat (EDTA),
auf oder besteht daraus
und ist insbesondere frei von Reduktionsmitteln, z.B. frei von Thiolverbindungen oder deren Vorläuferverbindungen, insbesondere frei von β-Mercaptoethanol und Dithiothreitol (DTT).

In wässriger Lösung besteht die Zusammensetzung aus dem zumindest einen Guanidiniumsalz z.B. zu 1,8 bis 2,6 M, bevorzugt 2,0 bis 2,4 M, bevorzugter 2,2 M auf,
der zumindest einen Puffersubstanz in einer Konzentration, die ein Blutvolumen auf einen pH von 5,0 bis 8,0, z.B. von pH 5,1, 5,2, 5,3, 5,4, 5,5, 5,6, 5,7, 5,8, 5,9 oder 6,0 bis 7,5, 7,6, 7,7, 7,8, 7,9, bevorzugt pH 6,5 bis 7,3 puffert, z.B. MES zu zumindest 50 mM, bevorzugt zumindest 70 mM, z.B. bis 100 mM oder bis 74 mM,
dem nichtionischen Detergenz, z.B. Triton X-100, zu 10 bis 20 % Gew./Vol., bevorzugt 12 bis 18 % Gew./Vol.,
einem Komplexbildner für zweiwertige Kationen, insbesondere EDTA, von z.B. 50 bis 100 mM, bevorzugt 70 bis 80 mM, bevorzugter ca. 72 mM,
z.B. für ein Blutvolumen bis zu einem Verhältnis zum Volumen der wässrigen Zusammensetzung von 1:13 oder 1:3 oder bis 1:2, bevorzugt bis 1:2,6 oder bis 1:2,5 oder 1:2,4.

Die Puffersubstanz ist z.B. ausgewählt aus Tris (Tris(hydroxymethyl)-aminomethan), Citrat oder Phosphat(di-Natriumhydrogenphosphat)dihydrat bzw. Phosphat-(Natriumhydrogenphosphat)dihydrat, BisTris-Puffer (Bis-(2-hydroxymethyl)-imino-tris-(hydroxymethyl)-methan), ACES (N-(2-Acetamido)-2-aminomethansulfonsäure), Natriumhydrogencarbonat, bevorzugt 2-(*N*-Morpholino)ethansulfonsäure (MES).

Das nichtionische Detergenz ist z.B. ausgewählt aus Tween 20 (Polyoxyethylen(20)-sorbitanmonolaurat), Nonidet P40 (4-Nonylphenol-polyethylenglycol), Tween 80 (Polyoxyethylensorbitanmonooleat), Brij 58 (Polyethylenglycol-hexadecylether), Triton X-114 (Octylphenolpolyethylenglycolether), bevorzugt Triton X-100 (Polyethylenglycol-p-(1,1,3,3-tetramethylbutyl)phenylether).

Die Zusammensetzung hat den Vorteil, die in Vollblut enthaltenen menschlichen Zellen aufzuschließen und die gesamten Nukleinsäuren, insbesondere RNA, aus Vollblut zu stabilisieren, wobei Vollblut und die Zusammensetzung in einem variablen Volumenverhältnis vorliegen können. Eine Blutentnahmeröhre, die die Zusammensetzung enthält, kann daher eingerichtet sein, ein variables Blutvolumen einzuziehen, z.B. kann die Blutentnahmeröhre eingerichtet sein, ein Blut bis zu einem volumetrischen Verhältnis von 1:13 oder 1:3, bevorzugt bis 1:2 oder bis 1:2,6 zu der Zusammensetzung einzuziehen. Für das Einziehen eines variablen Blutvolumens kann die Blutentnahmeröhre, in der die Zusammensetzung enthalten ist, z.B. einen manuell aus einer Röhre herausziehbaren Kolben aufweisen.

Die Zusammensetzung zeichnet sich dadurch aus, dass sie keine Ausfällung der Nukleinsäuren aus der Mischung aus Vollblut und der Zusammensetzung erfordert, um die RNA aus dem Vollblut zu stabilisieren und zu isolieren. Daher weist das erfindungsgemäße Verfahren das Isolieren von RNA aus der Mischung aus Vollblut und der Zusammensetzung auf, ohne einen Schritt des Fällens von Nukleinsäuren, insbesondere RNA, beziehungsweise ohne einen Schritt des Zentrifugierens zur Abtrennung der ausgefällten Nukleinsäuren vor der Bindung der RNA an ein Adsorptionsmittel.

Erfindungsgemäß bevorzugt wird die gesamte RNA aus der Mischung aus Vollblut und der Zusammensetzung dadurch isoliert, dass die Mischung aus Vollblut und der Zusammensetzung,
optional nach Zusetzen von Proteinase, z.B. Proteinase K und Inkubieren, z.B. bei Raumtemperatur für ca. 15 min, optional vor oder nach Zusetzen der Proteinase Zusetzen von DNase,
mit einem Adsorptionsmittel für Nukleinsäuren kontaktiert wird,
wobei von der Mischung, optional einschließlich zugesetzter Proteinase und/oder DNase, vor dem Kontaktieren mit dem Adsorptionsmittel kein Inhaltsstoff abgetrennt wird, anschließend die nicht an das Adsorptionsmittel gebundenen Stoffe entfernt werden, z.B. durch Waschen des Adsorptionsmittels,
und an das Adsorptionsmittel gebundene Nukleinsäuren eluiert werden, z.B. durch Kontaktieren des Adsorptionsmittels mit einem wässrigen Puffer mit einem pH-Wert, einem Gehalt an Alkohol und/oder einer Ionenkonzentration, die an das Adsorptionsmittel gebundene Nukleinsäuren löst.

Die Mischung aus Vollblut und der Zusammensetzung kann vollständig und zeitlich direkt nach Herstellen der Mischung mit dem Adsorptionsmittel für Nukleinsäuren kontaktiert werden. Daraus ergibt sich der Vorteil, dass das Verfahren zur Isolierung der RNA keine Inkubation zur Fällung und keine Zentrifugation vor der
Kontaktierung der Mischung mit dem Adsorptionsmittel erfordert und keinen Zusatz weiterer Stoffe und daher einfach und schnell durchführbar ist.

Nach dem Kontaktieren der Mischung aus Vollblut und der Zusammensetzung, die optional zugesetzte Proteinase enthält, kann optional DNase zugesetzt werden, so dass DNA verdaut wird und im Wesentlichen RNA isoliert wird.

Das Adsorptionsmittel für Nukleinsäuren ist z.B. eine Silikaoberfläche, insbesondere Silika(gel)membran, Silika(gel)suspension oder silikabeschichtete Magnetpartikel wie es z.B. unter der Bezeichnung PAXgene der Firma PreAnalytiX, der Bezeichnung NucleoSpin von der Firma Macherey-Nagel, der Bezeichnung mRNA Isolation Kit for Blood/Bone Marrow oder der Bezeichnung High Pure Viral Nucleic Acid Large Volume Kit der Firma Roche zur Isolierung von Nukleinsäuren erhältlich ist.

Wenn das Adsorptionsmittel aus silikabeschichteten Partikeln, bevorzugt Magnetpartikeln, besteht, ist der Zusatz eines Verdünners in effektivem Volumen zu der Mischung aus Vollblut und der Zusammensetzung bevorzugt. Der Verdünner kann z.B. das von der Fa. Roche erhältliche DNA/RNA-Stabilization Reagent for Blood/Bone marrow (Roche Artikel Nr. 11 934 317 001) sein oder die erfindungsgemäße Zusammensetzung, jeweils optional mit einem Gehalt an bis zu 30 Vol./Vol.-% Ethanol, bevorzugt 10 bis 30 Vol./Vol.-% Ethanol, z.B. 20 Vol./Vol.-% Ethanol. Ein effektives Volumen kann als solches bestimmt werden, bei dem die RNA im Wesentlichen zumindest zu 80% der Gesamtmenge, bevorzugt im Wesentlichen vollständig an die silikabeschichteten Partikel adsorbiert wird. Das effektive Volumen, mit dem der Verdünner zu der Mischung aus Vollblut und der Zusammensetzung gegeben wird, beträgt vorzugsweise 50 bis 150 % des Volumens der Mischung, z.B. 80 bis 120 %, bevorzugt 100 % des Volumens der Mischung, zugegeben vor oder nach dem Zumischen der silikabeschichteten Partikel in die Mischung, die vorzugsweise Magnetpartikel sind. Insbesondere in dieser Ausführungsform ist der Zusatz von Proteinase zu der Mischung bevorzugt.

Silikabeschichtete Partikel können z.B. solche der Firma Chemagen oder der Firma Applied Biosystems (RNA binding beads, Art.-Nr. 100191) oder der Firma Roche aus dem mRNA Isolation Kit for Blood/Bone Marrow, Art.-Nr. 11 934 333 001 sein oder solche gemäß Hai N.H., Phu N.D., Luong N.H., Chau N., Chinh H.D., Hoang L.H., Leslie-Pelecky D.L. (2008), Mechanism for Sustainable Magnetic Nanoparticles under Ambient Conditions, Journal of the Korean Physical Society, 52 (5), 1327-1331 oder solche gemäß Quy D.V., Hieu N.M., Tra P.T., Nam N.H., Hai N.H., Son N.T., Nghia P.T., Anh N.T.V., Hong T.T., Luong N.H. (2013) Synthesis of Silica-Coated Magnetic Nanoparticles and Application in the Detection of Pathogenic Viruses, Journal of Nanomaterials, DOI: 10.1155/2013/603940.

Auch bei einem Adsorptionsmittel aus silikabeschichteten Partikeln, die bevorzugt silikabeschichtete Magnetpartikel sind, wird die vollständige Mischung aus Vollblut und der Zusammensetzung mit dem Adsorptionsmittel mit dem zugesetzten Verdünner kontaktiert, also unmittelbar bzw. vollständig bzw. ohne vorheriges Abtrennen eines Bestandteils der Mischung.

Erfindungsgemäß kann die vollständige Mischung unmittelbar mit dem Adsorptionsmittel kontaktiert werden, da aus der Mischung aus Vollblut und der Zusammensetzung kein Inhaltsstoff bzw. keine Fraktion abgetrennt wird, insbesondere ist keine Zentrifugation dieser Mischung zur Abtrennung eines ausgefällten Inhaltsstoffs, z.B. kein Abzentrifugieren von ausgefällten Nukleinsäuren mit anschließendem Abtrennen der Flüssigphase und Lösen der Nukleinsäuren erforderlich. Dies wird gegenwärtig darauf zurückgeführt, dass die Zusammensetzung Nukleinsäuren, insbesondere RNA, in Mischung mit Vollblut nicht ausfällt. Die Zusammensetzung hat vielmehr den Vorteil, dass eine Mischung aus Vollblut und der Zusammensetzung, optional mit zugegebener Proteinase und/oder DNase, ohne weitere Behandlungsschritte bzw. ohne Zusätze mit einem Adsorptionsmittel für Nukleinsäuren, insbesondere Silikaoberflächen, kontaktiert werden kann, um die Nukleinsäuren an das Adsorptionsmittel zu binden. Daher erlaubt die Zusammensetzung ein Verfahren zur Isolierung von Nukleinsäuren, das ohne Abtrennung einer Nukleinsäuren enthaltenden Fraktion vor dem Kontaktieren mit dem Adsorptionsmittel durchgeführt wird, z.B. ohne Fällung mittels Zentrifugation mit anschließendem Lösen.

Die Zusammensetzung stabilisiert die RNA in der Mischung aus Vollblut und der Zusammensetzung, z.B. bei einer Lagerung für zumindest 3 Tage, bevorzugt für zumindest 5 Tage, z.B. für bis zu 4 Tage, insbesondere ohne Einfrieren, z.B. Lagerung bei zumindest 0°C, z.B. bei Raumtemperatur bis 22,5 °C. Entsprechend weist das Verfahren bevorzugt die Isolierung der RNA aus der Mischung auf, z.B. nach einer Lagerung ohne Einfrieren bzw. bei zumindest 0°C, z.B. bei 15 bis 25 °C,
insbesondere bei bis zu 22,5°C. Alternativ oder zusätzlich kann die Mischung zur Lagerung eingefroren werden, z.B. bei -40°C oder darunter.

Die Erfindung wird nun genauer anhand von Beispielen mit Bezug auf die Figuren beschrieben, die in
- Figur 1 die Integrität der RNA, die aus Vollblut in Mischung mit einer erfindungsgemäßen Zusammensetzung in verschiedenen volumetrischen Verhältnissen isoliert wurden,
- Figur 2 die Konzentrationen der RNA, die aus Vollblut in Mischung mit einer erfindungsgemäßen Zusammensetzung in verschiedenen volumetrischen Verhältnissen isoliert wurden,
- Figur 3 und 4 der Vergleich der RNA-Integrität aus Vollblutproben (Sp. 1, Spender Nr. 1 und Sp. 2, Spender Nr. 2) nach Lagerung in einem Stabilisator gemäß WO 00/09746 gegenüber einer Lagerung in einer erfindungsgemäßen Zusammensetzung, Figur 5 eine Clusteranalyse der Konzentration verschiedener mRNAs in der gesamten RNA, die aus Vollblut in Mischung mit einer erfindungsgemäßen Zusammensetzung isoliert wurde,
- Figur 6 eine Korrelationsmatrix zur Darstellung von lagerungsabhängigen Expressionsunterschieden,
- Figuren 7 und 8 die relativen Mengen mittels RT-PCR bestimmter mRNAs in Proben zweier Spender zeigen, die nach Lagerung von Vollblut über 5 Tage in Mischung mit einer erfindungsgemäßen Zusammensetzung isoliert wurden.

Die erfindungsgemäße Zusammensetzung wurde als wässrige Lösung in einer Blutentnahmeröhre vorgelegt, in die eine Blutprobe eingezogen wurde. Die Blutentnahmeröhre kann direkt zur Entnahme von Blut verwendet werden. Alternativ kann die Zusammensetzung mit Vollblut gemischt werden, das bevorzugt aus einer direkt zuvor entnommenen Vollblutprobe stammt. Der Aufschluss von Zellen der Blutprobe und die Stabilisierung der Nukleinsäuren, insbesondere der RNA, erfolgt generell durch das Mischen der Vollblutprobe mit der Zusammensetzung.

### Beispiel 1: Isolierung von RNA aus Vollblut

Als erfindungsgemäße Zusammensetzung wurde eine wässrige Lösung verwendet, die aus 2,2 M Guanidiniumthiocyanat, 72 mM MES, 14,4 % (Gew./Vol.) Triton X-100 und 72 mM EDTA in Wasser bestand. Von dieser Zusammensetzung waren 6,5 mL in einer herkömmlichen Blutentnahmeröhre enthalten, in die direkt zuvor entnommenes Blut aufgezogen wurde, das nicht weiter behandelt war und keinen Zusatz enthielt. Dieses Vollblut wurde in einem Volumen von 0,5 mL, 1,0 mL, 1,5 mL, 2,0 mL und 2,5 mL in jeweils eine Blutentnahmeröhre aufgezogen. Die Durchmischung erfolgt durch das Aufziehen des Blutvolumens, optional zusätzlich durch Schütteln, insbesondere nach Einziehen einer Luftblase.

Die Mischung aus der Zusammensetzung und Vollblut wurde mit Proteinase K (250 µL, 18 µg/mL) versetzt. Die Mischung wurde ca. 15 min bei Raumtemperatur inkubiert. Anschließend wurde die Mischung vollständig durch eine Säule mit Adsorptionsmittel (High Pure Viral Nucleic Acid Large Volume Kit, erhältlich von Roche) für Nukleinsäuren gegeben. Das Adsorptionsmittel wurde mit Waschpuffer gemäß den Anweisungen des Herstellers gewaschen, anschließend wurden gebundene Nukleinsäuren mit 100µL Elutionspuffer eluiert.

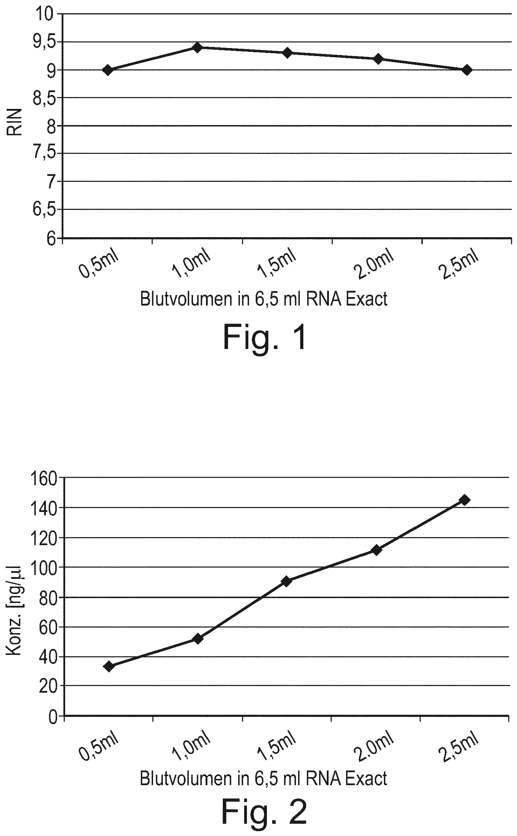

Die Qualität der eluierten RNA wurden mittels Elektrophorese in einem RNA Nano Chipsystem (Agilent Technologies, USA), Detektion mittels Agilent 2100 Bioanalyser bewertet, wobei eine so genannte RIN (RNA Integrity Number), die in der Analyse mittels Bioanalyzer 2100 ermittelt wird, als Maß für die RNA-Integrität angegeben wird. Figur 1 zeigt mit dem Ergebnis der Analyse für die verschiedenen Volumenverhältnisse von Vollblut zu Zusammensetzung (RNA Exact), dass die RNA-Integrität nicht von dem Volumenverhältnis abhängt, bzw. dass die Zusammensetzung RNA gleicher Qualität für verschiedene Volumenverhältnisse mit Vollblut stabilisiert und die RNA aus deren Mischung isoliert werden kann.

Figur 2 zeigt die gemessene RNA-Konzentration im Eluat, die für die verschiedenen Volumenverhältnisse isoliert wurden. Die Ergebnisse zeigen durch die proportional zum Blutvolumen steigende RNA-Konzentration in 100 µL Eluat, dass die Zusammensetzung (RNA Exact) die RNA im gleichen Ausmaß für die verschiedenen Volumenverhältnisse stabilisiert und daraus im gleichen Ausmaß isoliert wird. Dies zeigt, dass die Zusammensetzung für die Lyse von Zellen in Vollblut und die Stabilisierung der Nukleinsäuren in Vollblut in verschiedenen Volumenverhältnissen geeignet ist.

### Beispiel 2: Vergleich der Stabilisierungsleistung der erfindungsgemäßen Zusammensetzung mit einer Vergleichslösung.

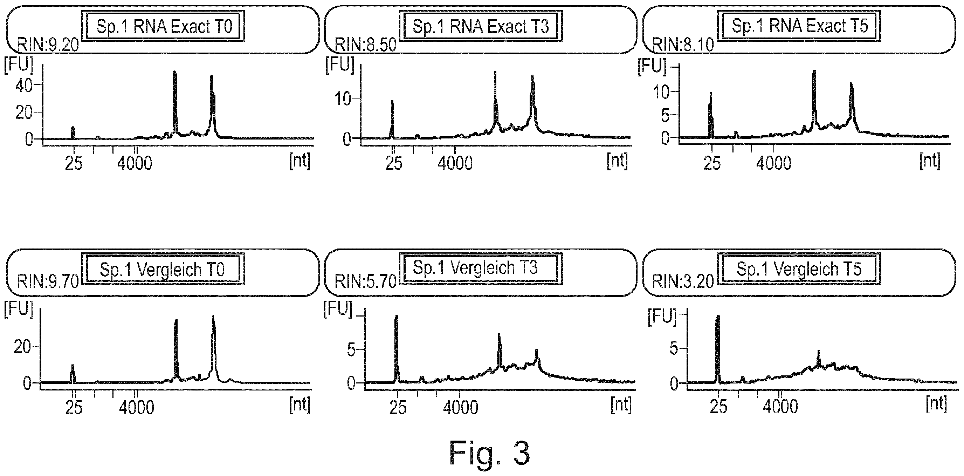

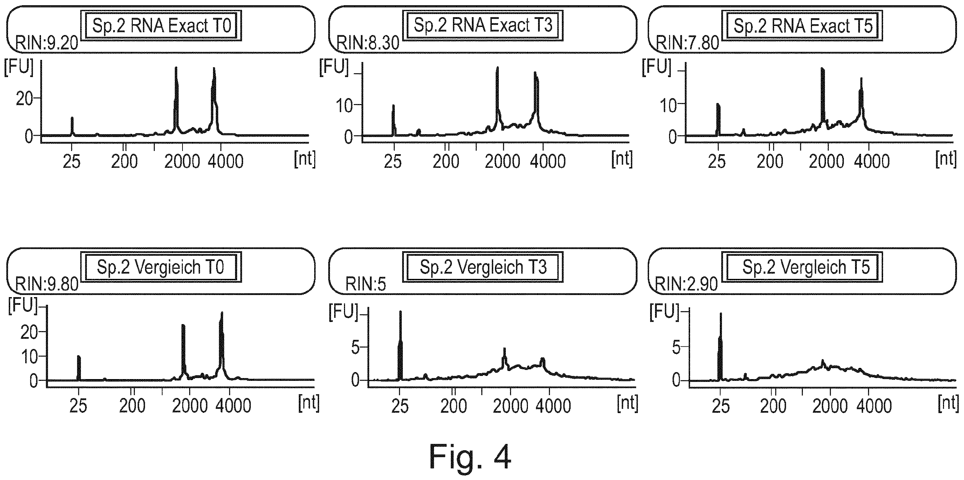

Als erfindungsgemäße Zusammensetzung (RNA Exact) wurde diejenige von Beispiel 1 zu je 6,5 mL verwendet und mit 2,5 mL direkt zuvor entnommenem Blut gemischt, das keine anderen Zusatzstoffe enthielt, um die Mischung aus der Zusammensetzung und Vollblut herzustellen. Zum Vergleich wurde eine Lösung verwendet, die aus 4,0 M Guanidiniumthiocyanat, 45 mM Tris/HCL, 18,0 % (Gew./Vol.) Triton X-100 und 0,8 % (Gew./Vol.) DTT in Wasser bestand und auf einen pH-Wert von 6,0 eingestellt war (Vergleich, entsprechend WO 00/09746 A1). 2,5 ml der Vergleichslösung wurden, wie in WO 00/09746 A1 beschrieben (Verhältnis 1+1), mit ebenfalls 2,5 ml der direkt zuvor entnommenen Blutprobe vermischt. Die Mischungen wurden bei 22,5°C gelagert und die RNA wurde mit dem NucleoSpin RNA Blood Midi Kit der Fa. Macherey-Nagel aus den Mischungen isoliert. In die Vergleichslösung wurde, wie im Protokoll von Macherey-Nagel vorgesehen, 1/3 des Volumens an 70 % Ethanol zugesetzt. Für die Mischung mit der erfindungsgemäßen Zusammensetzung ist dies nicht notwendig. Die Figur 3 und die Figur 4 zeigen die Ergebnisse der kapillarelektrophoretischen Auftrennung der Eluate auf einem RNA Nano-Chip im Bioanalyzer 2100. Während aus dem Probengemisch mit der erfindungsgemäßen Zusammensetzung auch nach 3 Tagen und nach 5 Tagen RNA mit hoher Integrität isoliert werden kann, ist dies unter Verwendung der Vergleichslösung nur am Tag 0, also direkt nach der Vermischung mit Blut möglich.

### Beispiel 3: Stabilisierung der RNA aus Vollblut und Isolierung

Als erfindungsgemäße Zusammensetzung wurde diejenige von Beispiel 1 zu je 6,5 mL verwendet und mit 2,5 mL direkt zuvor entnommenem Blut gemischt, das keine anderen Zusatzstoffe enthielt, um die Mischung aus der Zusammensetzung und Vollblut herzustellen. Zum Vergleich wurde Blut in Blutentnahmeröhrchen aufgezogen, die EDTA zur Hemmung der Coagulation enthielten (S-Monovetten EDTA-K3, erhältlich von der Firma Sarstedt). Das Blut stammte von jeweils drei verschiedenen Spendern.

Die erfindungsgemäßen Mischungen und die Vergleichsproben wurden bei 22,5 °C inkubiert, wobei ein Aliquot sofort entnommen wurde (T0, Tag Null), nach 1 Tag Inkubation (T1) und nach 3 Tagen Inkubation (T3). Aus den entnommenen Aliquots wurde unmittelbar und sofort die Nukleinsäure isoliert, wie in Beispiel 1 beschrieben.

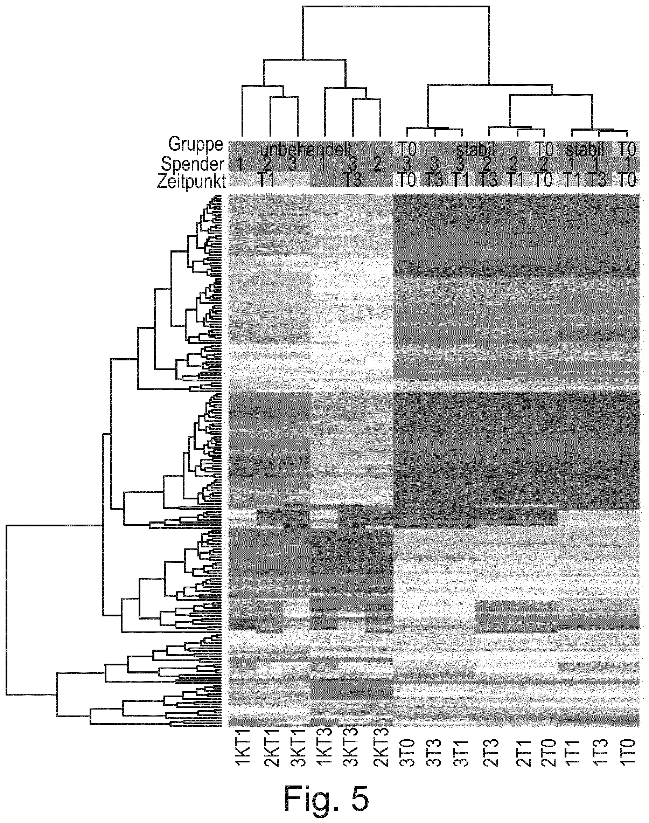

Die eluierten Nukleinsäuren wurden zur Entfernung von DNA mit DNase (Thermo Fisher Scientific, Artikel Nr. EN0521) behandelt und mittels einer Mikroarray-Analyse auf die Konzentration von ca. 47000 Transkripten (mRNA) getestet (humanHT-12 v4.0 Expression Bead Chip der Fa. Agilent). Bei der Auswertung werden erst Unterschiede der Menge an Transkripten um mehr als den Faktor 2 bei gleicher Gesamtmenge RNA angezeigt. In Fig. 5 zeigen daher gleiche Helligkeiten in der Matrix vergleichbare Transkriptmengen. Die Fig. 5 zeigt die Transkriptmengen für die Probengruppen Stabil (erfindungsgemäße Zusammensetzung), unbeh (nur mit EDTA versetzt) und T0 (ohne Zusatz, sofort nach Blutentnahme isoliert) jeweils für die Proben der Spender 1, 2 und 3 zu den angegebenen Zeitpunkten.

Die Aliquots, die in Figur 5 analysiert sind, sind

| Spender | 1 | | 2 | | 3 | |
|---|---|---|---|---|---|---|
| Probe | 1T0 | | 2T0 | | 3T0 | |
| Probe | 1T1 | 1KT1 | 2T1 | 2KT1 | 3T1 | 3KT1 |
| Probe | 1T3 | 1KT3 | 2T3 | 2KT3 | 3T3 | 3KT3 |

wobei EDTA-Vergleichsproben mit "K" gekennzeichnet sind.

Die Clusteranalyse an 199 beispielhaft ausgewählten Transkripten, in Figur 5 gezeigt, macht deutlich, dass die Blutproben, die mit der erfindungsgemäßen Zusammensetzung (Stabil) gemischt wurden, für einen Spender zu den verschiedenen Zeitpunkten der Inkubation jeweils ein vergleichbares Muster von Transkripten zeigen, während die Vergleichsproben (unbeh), die nur mit EDTA versetzt waren, zu den verschiedenen Zeitpunkten der Inkubation große Unterschiede innerhalb jedes Spenders zeigten, was auf eine Veränderung der Transkriptmengen über die Zeit und gegenüber T0 hinweist.

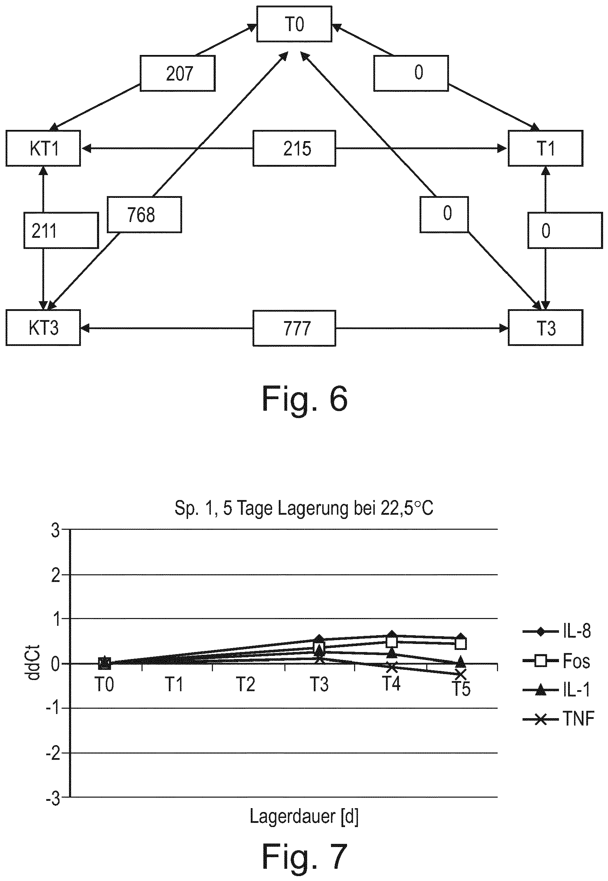

Für die nur mit EDTA versetzten Blutproben wurden an Tag 3 gegenüber Tag 0 für 768 Transkripte Unterschiede in der Konzentration gefunden, wohingegen bei den Proben, die mit der erfindungsgemäßen Zusammensetzung versetzt waren, innerhalb jedes Spenders wesentlich geringere bis keine Unterschiede in der Konzentration der individuellen RNAs der zu späteren Zeitpunkten analysierten Proben im Vergleich zu T0 gefunden wurden (siehe Korrelationsmatrix von Figur 6, Vergleichsproben = K, jeweils zu Zeitpunkten als KT0, KT1, KT3, mit erfindungsgemäßer Zusammensetzung T0, T1, T3).

Dieses Ergebnis zeigt anhand der konstanten Mengen für Transkripte für einzelne Spender über die Zeit von 3 Tagen in den mit der Zusammensetzung gemischten Aliquots der Blutproben, dass die Zusammensetzung der Erfindung die individuellen RNA-Moleküle aus Vollblut über die Inkubation stabilisiert und dass die Zusammensetzung z.B. im Vergleich mit den EDTA-Vergleichsproben die Degeneration und/oder Induktion von mRNA signifikant vermindert.

### Beispiel 4: Reverse Transkription und PCR auf Basis der RNA aus Vollblut

Von zwei verschiedenen Spendern stammendes Vollblut wurde zu je 2,5 mL direkt nach der Entnahme mit 6,5 mL der erfindungsgemäßen Zusammensetzung aus 2,2 M Guanidiniumthiocyanat, 72 mM MES, 14,4 % (Gew./Vol.) Triton X-100 und 72 mM EDTA in Wasser versetzt und bei 22,5 °C inkubiert. Aus diesen Mischungen wurde sofort (T0) und nach 3 Tagen (T3), nach 4 Tagen (T4) und nach 5 Tagen (T5) jeweils Aliquots entnommen und bei -80°C eingefroren. Anschließend wurden alle Proben parallel aufgetaut und auf Nukleinsäure-Adsorptionsmittel (NucleoSpin RNA Blood, Macherey-Nagel) gegeben. Das Adsorptionsmittel wurde gemäß den Anweisungen des Herstellers gewaschen und die Nukleinsäuren wurden eluiert. Dem Eluat wurde DNase (DNase I, RNase frei, Artikel Nr. EN0521, Thermo Fisher Scientific) zugesetzt. Die RNA wurde mit dem "first strand cDNA synthesis kit" (Artikel Nr. K1612, Thermo Fisher Scientific) revers transkribiert, um cDNA zu erhalten. Dieses Produkt wurde mit Primern, die für die Nukleotidsequenzen der Gene für β-Actin, GAPDH, IL-8, c-Fos, IL-1B und TNF-α spezifisch waren, mittels real-time PCR, unter Verwendung des Maxima SYBR Green/ROX qPCR Master Mix (Artikel Nr. #K0222, Thermo Fisher Scientific) als Doppelansatz amplifiziert und quantifiziert.

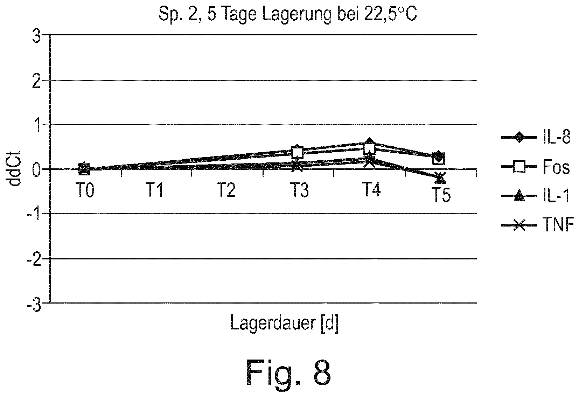

Zur Auswertung wurden die für die cDNA von β-Actin und GAPDH erhaltenen Ct-Werte als interner Standard verwendet und darauf die für IL-8, c-Fos, IL-1B und TNF-α gemessenen Ct-Werte normalisiert. Diese relativen Konzentrationen der cDNAs für IL-8, c-Fos, IL-1B und TNF-α bei T3, T4 und T5 wurden zusätzlich auf ihre jeweilige Konzentration bei T0 bezogen. Diese Ergebnisse werden als ddCt bezeichnet und sind in Figur 7 für das Blut des einen Spenders, in Figur 8 für das Blut des anderen Spenders gezeigt. Die Ergebnisse beider Proben machen deutlich, dass die erfindungsgemäße Zusammensetzung die relativen Konzentrationen der spezifisch detektierten zellulären mRNAs in Vollblut über die Inkubationsdauer bei 22,5 °C stabil erhält.

## Patentansprüche

1. Verwendung einer Zusammensetzung als Aufschlussmittel für in Vollblut enthaltene Zellen, als Stabilisierungsmittel für die gesamte RNA des Vollbluts und zur Isolierung mittels Adsorption der RNA aus einer Mischung des Vollbluts mit der Zusammensetzung an ein Adsorptionsmittel für Nukleinsäuren, **dadurch gekennzeichnet, dass** die Zusammensetzung in wässriger Lösung aus
a. zumindest einem Guanidiniumsalz,
b. zumindest einer Puffersubstanz zum Puffern in einen pH von 5,0 bis 8,0,
c. zumindest einem nichtionischen Detergenz und
d. zumindest einem Komplexbildner für zweiwertige Kationen
e. und optional Proteinase und/oder DNase besteht und
f. frei von Reduktionsmitteln ist,
die den Abbau und die Neusynthese von RNA im Vollblut verhindert, und dass die Verwendung die Isolierung von RNA unmittelbar aus der Mischung umfasst, die aus Vollblut und der Zusammensetzung besteht, wobei die Zusammensetzung in einem variablen Volumenverhältnis des Vollbluts zur Zusammensetzung von bis zu 1:13 in der Mischung vorgesehen ist, mittels Kontaktieren der Mischung mit einem Adsorptionsmittel für Nukleinsäuren, wobei aus der Mischung kein Bestandteil abgetrennt wird, optional der Mischung Proteinase und/oder DNase zugesetzt wird, und die RNA nicht aus der Mischung ausgefällt und abgetrennt wird, bevor sie mit dem Adsorptionsmittel kontaktiert wird.

2. Verwendung nach Anspruch 1, **dadurch gekennzeichnet, dass** das Guanidiumsalz Guanidiniumthiocyanat, die Puffersubstanz 2-(*N*-Morpholino)ethansulfonsäure (MES), Tris (Tris(hydroxymethyl)-aminomethan), Citrat, Phosphat(di-Natriumhydrogenphosphat)dihydrat, BisTris-Puffer (Bis-(2-hydroxymethyl)-imino-tris-(hydroxymethyl)-methan), ACES (N-(2-Acetamido)-2-aminomethansulfonsäure), Natriumhydrogencarbonat oder Phosphat-(Natriumhydrogenphosphat)dihydrat ist, das nichtionische Detergenz Triton X-100, Tween 20, Tween 80 (Polyoxyethylensorbitanmonooleat), Brij 58 (Polyethylenglycol-hexadecylether), Triton X-114 (Octylphenolpolyethylenglycolether) oder Nonidet P40 ist und der Komplexbildner Ethylendiamintetraacetat (EDTA) ist und sie frei ist von Thiolverbindungen oder deren Vorläuferverbindungen als Reduktionsmittel.

3. Verwendung nach einem der voranstehenden Ansprüche, **gekennzeichnet dadurch, dass** die Zusammensetzung in einer Blutentnahmeröhre enthalten ist.

4. Verwendung nach Anspruch 3, **gekennzeichnet dadurch, dass** die Blutentnahmeröhre eingerichtet ist, ein variables Volumen des Vollbluts aufzuziehen.

5. Verfahren zur Isolierung der gesamten RNA aus Vollblut, **gekennzeichnet durch** Mischen des Vollbluts mit einer Zusammensetzung, die in wässriger Lösung aus
a. zumindest einem Guanidiniumsalz,
b. zumindest einer Puffersubstanz,
c. zumindest einem nichtionischen Detergenz und
d. zumindest einem Komplexbildner,
e. optional Proteinase und/oder DNase
besteht und frei von Reduktionsmitteln ist, die den Abbau und die Neusynthese von RNA im Vollblut verhindert, Herstellen einer Mischung, die aus dem Vollblut und der Zusammensetzung in einem Volumenverhältnis von bis zu 1:13 besteht, optional mit Lagerung der erhaltenen Mischung bei oberhalb 0°C, optional mit Zugabe von Proteinase und/oder DNase zu der Mischung, Kontaktieren der Mischung mit einem Adsorptionsmittel für Nukleinsäuren, wobei von der Mischung vor dem Kontaktieren mit dem Adsorptionsmittel kein Inhaltsstoff abgetrennt wird, Entfernen von ungebundenen Stoffen von dem Adsorptionsmittel, Eluieren von RNA von dem Adsorptionsmittel.

6. Verfahren nach Anspruch 5, **dadurch gekennzeichnet, dass** das Kontaktieren der Mischung mit dem Adsorptionsmittel ohne vorheriges Ausfällen, Abtrennen und Lösen von Nukleinsäuren aus der Mischung erfolgt.

7. Verfahren nach einem der Ansprüche 5 bis 6, **dadurch gekennzeichnet, dass** das Lagern der Mischung für zumindest 3 Tage bei maximal 25°C erfolgt.

8. Verfahren nach einem der Ansprüche 5 bis 7, **dadurch gekennzeichnet, dass** das Mischen des Vollbluts mit der Zusammensetzung in einem Volumenverhältnis von bis zu 1:2 erfolgt.

9. Verfahren nach einem der Ansprüche 5 bis 8, **dadurch gekennzeichnet, dass** das Adsorptionsmittel silikabeschichtete Partikel sind und der Mischung des Vollbluts und der Zusammensetzung ein Verdünner in einem Volumen von zumindest 50 % des Volumens der Mischung zugesetzt wird.

## Claims

1. Use of a composition as a lysis agent for cells contained in whole blood as a stabilising agent for the total RNA of the whole blood and for isolating RNA by means of adsorption from a mixture of the whole blood with the composition to an adsorption agent for nucleic acids, **characterized in that** the composition in aqueous solution consists of
a. at least one guanidinium salt,
b. at least one buffering agent for buffering to a pH of 5.0 to 8.0,
c. at least one nonionic detergent and
d. at least one complexing agent for divalent cations,
e. and optionally proteinase and/or DNase, and
f. is free from reduction agents,
which prevents the degradation and novel synthesis of RNA in whole blood, and by the use comprising the isolation of RNA directly out of the mixture, which consists of the whole blood and the composition, wherein the composition is provided in a variable volumetric relation of the whole blood to the composition of up to 1:13 in the mixture, by means of contacting the mixture with an adsorption agent for nucleic acids, wherein no component is separated from the mixture, optionally proteinase and/or DNase is added to the mixture, and the RNA is not precipitated and separated from the mixture prior to contacting it with the adsorption agent.

2. Use according to claim 1, **characterized in that** the guanidinium salt is guanidiniumthiocyanate, the buffering agent is 2-(N-morpholino)ethanesulfonic acid (MES), Tris (tris(hydroxymethyl)-aminomethane), citrate, phosphate(di-sodium hydrogenphosphate)dihydrate, BisTrisbuffer (bis-(2-hydroxymethyl)-imino-tris-(hydroxymethyl)-methane), ACES (N-(2-acet amido)- 2-aminomethane sulfonic acid, sodium hydrogencarbonate or phosphate(sodium hydrogen phosphate)dihydrate, the nonionic detergent is Triton X-100, Tween 20, Tween 80 (polyoxyethylene sorbitanmonooleat), Brij 58 (polyethyleneglycol-hexadecylether), Triton X-114 (octylphenolpolyethylene glycolether) or Nonidet P40, and the complexing agent is ethylenediamine tetraacetate (EDTA) and it is free from the thiol compounds or their precursor compounds as reduction agents.

3. Use according to one of the preceding claims, **characterized in that** the composition is contained in a blood drawing tube.

4. Use according to claim 3, characterized that the blood drawing tube is arranged to draw a variable volume of the whole blood.

5. Process for isolating the total RNA from whole blood, **characterized by** mixing the whole blood with a composition which consists of
a. at least one guanidinium salt,
b. at least one buffering agent,
c. at least one nonionic detergent and
d. at least one complexing agent,
e. optionally proteinase and/or DNase
in aqueous solution and is free from reduction agents, which prevents the degradation and novel synthesis of RNA in whole blood, producing a mixture consisting of the whole blood and the composition in a volumetric ratio of up to 1:13, optionally with storing the obtained mixture at above 0°C, optionally with addition of proteinase and/or DNase to the mixture, contacting the mixture with an adsorption agent for nucleic acids, wherein from the mixture prior to contacting the adsorption agent no ingredient is separated, removing unbound compounds from the adsorption agent, eluting of RNA from the adsorption agent.

6. Process according to claim 5, **characterized in that** the contacting of the mixture with an adsorption agent occurs without prior precipitation, separating and dissolving of nucleic acids from the mixture.

7. Process according to one of claims 5 to 6, **characterized in that** storing the mixture occurs for at least 3 days at at maximum 25 °C.

8. Process according to one of claims 5 to 7, **characterized in that** the mixing of the whole blood with the composition occurs in a volumetric ratio of up to 1:2.

9. Process according to one of claims 5 to 8, **characterized in that** the adsorption agent is silica - coated particles and that a thinner is added to the mixture of the whole blood and of the composition in a volume of at least 50% of the volume of the mixture.

## Revendications

1. Utilisation d'une composition comme agent désintégrant des cellules contenues dans le sang total, comme stabilisant pour l'ARN total du sang total et pour l'isolement par adsorption de l'ARN à partir d'un mélange du sang total avec la composition au niveau d'un adsorbant pour acides nucléiques, **caractérisé en ce que** la composition est une solution aqueuse composée des éléments suivants:
a. au moins un sel de guanidinium,
b. au moins une substance tampon pour tamponner à un pH de 5,0 à 8,0,
c. au moins un détergent non ionique et
d. au moins un agent complexant pour les cations divalents
e. et éventuellement une protéinase et/ou une DNase et
f. est exempt d'agents réducteurs,
qui empêche la dégradation et la re-synthèse de l'ARN dans le sang total et qui englobe l'isolement de l'ARN directement à partir du mélange comprenant du sang entier et la composition, dans laquelle la composition est prévue dans un rapport volumique variable du sang total à la composition allant jusqu'à 1:13 dans le mélange, en mettant en contact le mélange avec un adsorbant pour acides nucléiques, dans lequel aucun ingrédient n'est séparé du mélange, éventuellement ajouté au mélange de protéinase et/ou de DNase, et l'ARN n'est ni précipité du mélange ni séparé avant d'être mis en contact avec l'adsorbant.

2. Utilisation selon la revendication 1, **caractérisé en ce que** le sel de guanidium est du thiocyanate de guanidinium, l'agent tampon est l'acide éthansulfonique 2-(*N*-morpholino) (MES), un Tris (tris(hydroxyméthyle)aminométhane), un citrate, un dihydrate de phosphate (hydrogénophosphate disodique), un tampon BisTris (bis (2-hydroxyméthyle)-imino)- tris-(hydroxyméthyle)-méthane), un ACES (acide N-(2-acétamido)-2-aminométhanesulfonique), un bicarbonate de sodium ou un dihydrate de phosphate (hydrogénophosphate de sodium), le détergent non ionique est Triton X-100, Tween 20, Tween 80 (monooléate de polyoxyéthylène sorbitan) Brij 58 (polyéthylèneglycol hexadécyléther), Triton X114 (octylphénolpolyéthylene glycoléther) ou Nonidet P40 et l'agent complexant est l'éthylènediaminetétraacétate (EDTA) et il est exempt de composés thiols ou de leurs composés précurseurs en tant qu'agent réducteur.

3. Utilisation selon l'une des revendications précédentes, **caractérisée en ce que** la composition est contenue dans un tube de prélèvement sanguin.

4. Utilisation selon la revendication 3, **caractérisée en ce que** le tube de prélèvement sanguin est adapté pour faire croître un volume variable de sang total.

5. Procédé pour l'isolement de l'ARN total à partir de sang total, **caractérisé en ce que** le sang total est mélangé avec une composition qui, en solution aqueuse est composé des éléments suivants:
a. au moins un sel de guanidinium,
b. au moins une substance tampon,
c. au moins un détergent non ionique et
d. au moins un agent complexant,
e. éventuellement une protéinase et/ou un DNase
et est exempt d'agents réducteurs empêchant la dégradation et la resynthèse de l'ARN dans le sang total, produisant un mélange constitué du sang total et de la composition dans un rapport volumique allant jusqu'à 1:13, éventuellement avec stockage du mélange obtenu à plus de 0°C, éventuellement avec l'addition de protéinase et/ou de DNase au mélange, en mettant en contact le mélange avec un adsorbant pour acides nucléiques, aucun ingrédient n'étant séparé du mélange avant la mise en contact avec l'adsorbant, en éliminant les matières non liées de l'adsorbant, en éluant de l'ARN à partir de l'adsorbant.

6. Procédé selon la revendication 5, **caractérisé en ce que** la mise en contact du mélange avec l'adsorbant a lieu sans précipitation préalable, séparation préalable ni dissolution préalable des acides nucléiques du mélange.

7. Procédé selon l'une des revendications 5 à 6, **caractérisé en ce que** le mélange est stocké pendant au moins 3 jours à un maximum de 25°C.

8. Procédé selon l'une des revendications 5 à 7, **caractérisé en ce que** le mélange du sang total avec la composition s'effectue dans un rapport volumique allant jusqu'à 1:2.

9. Procédé selon l'une des revendications 5 à 8, **caractérisé en ce que** l'adsorbant consiste en particules enrobées de silice et qu'on ajoute au mélange de sang total et de la composition un diluant dans un volume d'au moins 50% du volume du mélange.
